# EUROPEAN PATENT SPECIFICATION

(11) **EP 3 946 551 B1**
(45) Date of publication and mention of the grant of the patent: **27.08.2025**
(21) Application number: 20722083.1
(22) Date of filing: 02.04.2020
(51) Int. Cl.: A61M 39/06

(54) **MULTI-USE BLOOD CONTROL CATHETER ASSEMBLY**
MEHRZWECK-BLUTKONTROLLKATHETER-ANORDNUNG
ENSEMBLE CATHÉTER DE CONTRÔLE SANGUIN MULTI-USAGE

(30) Priority: 04.04.2019 US 201962829458 P; 01.04.2020 US 202016837767
(43) Date of publication of application: 09.02.2022
(73) Proprietor: Becton, Dickinson and Company, Franklin Lakes, NJ 07417 (US)
(72) Inventor: TAGGE, Chad Alan, Sandy, Utah 84094 (US); MA, Yiping, Layton, Utah 84040 (US)
(74) Representative: dompatent von Kreisler Selting Werner - Partnerschaft von Patent- und Rechtsanwälten mbB
(86) International application number: PCT/US2020/026307
(87) International publication number: WO 2020/206064

(56) References cited:
- US-A- 4 512 766
- US-A- 5 330 435
- US-A1- 2011 160 662
- US-A1- 2018 256 872

## Description

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous catheter ("PIVC"). As its name implies, the over-the-needle PIVC may be mounted over an introducer needle having a sharp distal tip. The PIVC and the introducer needle may be assembled so that the distal tip of the introducer needle extends beyond the distal tip of the PIVC with the bevel of the needle facing up away from skin of the patient. The PIVC and introducer needle are generally inserted at a shallow angle through the skin into vasculature of the patient.

In order to verify proper placement of the introducer needle and/or the PIVC in the vein, a user generally confirms that there is "flashback" of blood in a flashback chamber of a PIVC assembly. Once placement of the needle has been confirmed, a user may temporarily occlude flow in the vein and remove the introducer needle, leaving the PIVC in place within the vein. The PIVC may then be used for fluid infusion and/or blood withdrawal or collection.

The subject matter claimed herein is not limited to embodiments that solve any disadvantages or that operate only in environments such as those described above. Rather, this background is only provided to illustrate one example technology area where some implementations described herein may be practiced. US 4512 766 A discloses a catheter assembly including a septum actuated by an actuator.

### SUMMARY

The present disclosure relates generally to catheter assemblies. The catheter assembly comprises a catheter adapter, comprising a distal end, a proximal end, and a lumen extending through the distal end and the proximal end; a septum disposed within the lumen, wherein the septum comprises a distal end, a proximal end, and a barrier disposed between the distal end of the septum and the proximal end of the septum, wherein the barrier divides an interior of the septum into a distal cavity and a proximal cavity, wherein the barrier comprises a slit, wherein the septum is configured to move from a proximal position to a distal position in response to insertion of a medical device into the proximal end of the catheter adapter; a spring disposed within the lumen distal to the septum, wherein the spring is configured to return the septum from the distal position to the proximal position in response to removal of the medical device from the proximal end of the catheter adapter, and a septum actuator fixed within the lumen and comprising a tubular body, wherein the septum actuator comprises an annular lip extending outwardly from an outer surface of the tubular body, wherein the annular lip is disposed at a proximal end of the septum actuator, wherein the septum actuator is configured to penetrate the slit in response to movement of the septum from the proximal position to the distal position,wherein an inner surface of the septum forming the distal cavity comprises a protrusion, wherein the annular lip is configured to pass the protrusion in response to the septum being moved from the proximal position to the distal position.

In some embodiments, the spring may surround the septum actuator.

In some embodiments, the spring and the septum may be constructed of an elastomer. For example, the spring and the septum may be constructed of silicon or another suitable material. In some embodiments, the spring and the septum may be monolithically formed as a single unit. In some embodiments, the spring may include a coil, which may surround the septum actuator. In some embodiments, the coil may be constructed of metal or another suitable material. In some embodiments, the coil may include a helical shape or multiple waves.

In some embodiments, an outer surface of the tubular body may be smooth. In some embodiments, the tubular body may be generally cylindrical or tapered.

In some embodiments, the annular lip may include a generally truncated cone shape.

In some embodiments, the one or more protrusions may be annular.

In some embodiments, a portion of the inner surface of the septum forming the distal cavity may be smooth.

It is to be understood that both the foregoing general description and the following detailed description are exemplary and explanatory and are not restrictive of the invention, as claimed. It should be understood that the various embodiments are not limited to the arrangements and instrumentality shown in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE SEVERAL VIEWS OF THE DRAWINGS

Example embodiments will be described and explained with additional specificity and detail through the use of the accompanying drawings in which:
Figure 1 is an upper perspective view of an example catheter system, according to some embodiments;
Figure 2A is a partial cutaway view of an example catheter assembly, illustrating an example spring and an example septum disposed in a proximal position, according to some embodiments;
Figure 2B is a cross-sectional view of the catheter assembly of Figure 2A, illustrating the spring and the septum disposed in the proximal position, according to some embodiments;
Figure 2C is a cross-sectional view of the catheter assembly of Figure 2A, illustrating the spring and the septum disposed in a distal position, according to some embodiments;
Figure 3A is a partial cutaway view of the catheter assembly, illustrating another example spring and the septum disposed in a proximal position, according to some embodiments;
Figure 3B is a cross-sectional view of the catheter assembly, illustrating the other spring and the septum disposed in the proximal position, according to some embodiments;
Figure 3C is a cross-sectional view of the catheter assembly, illustrating the other spring and the septum disposed in a distal position, according to some embodiments;
Figure 4A is a partial cutaway view of the catheter assembly, illustrating another example septum disposed in a proximal position, according to some embodiments;
Figure 4B is a cross-sectional view of the catheter assembly, illustrating the other septum disposed in a distal position, according to some embodiments;
Figure 5A is a partial cutaway view of the catheter assembly, illustrating the other septum disposed in a proximal position and an example septum actuator, according to some embodiments;
Figure 5B is a cross-sectional view of the catheter assembly, illustrating the other septum disposed in the proximal position and the septum actuator, according to some embodiments;
Figure 5C is a cross-sectional view of the catheter assembly, illustrating the septum other disposed in a distal position and the septum actuator, according to some embodiments; and
Figure 6 is an upper perspective view of the septum or the other septum, according to some embodiments.

### DESCRIPTION OF EMBODIMENTS

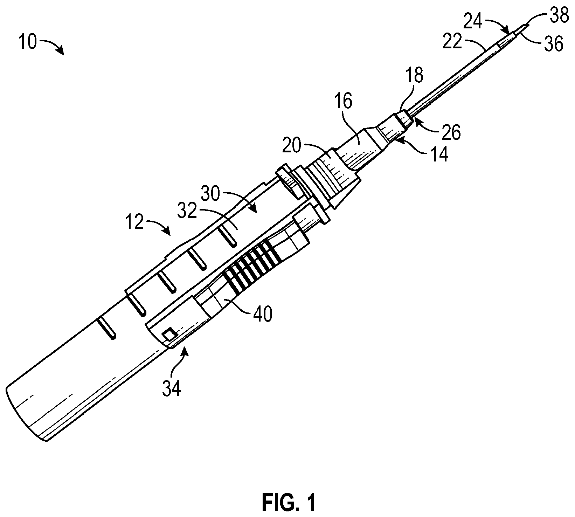

Referring now to Figure 1, a catheter system 10 is illustrated, according to some embodiments. In some embodiments, the catheter system 10 may include a needle shield device 12 and a catheter assembly 14. In some embodiments, the catheter assembly 14 may include a catheter adapter 16, which may include a distal end 18, a proximal end 20, and a lumen extending through the distal end 18 and the proximal end 20. In some embodiments, the catheter assembly 14 may include a catheter 22, which may include a distal end 24 and a proximal end 26. In some embodiments, the catheter 22 may include a peripheral intravenous catheter ("PIVC"), a midline catheter, a peripherally inserted central catheter ("PICC"), or another suitable catheter. In some embodiments, the proximal end 26 of the catheter 22 may be secured within the catheter adapter 16.

In some embodiments, the catheter system 10 may include one or more features of the safety catheter assembly described in U.S. Patent Application No. 15/012,013, filed February 1, 2016, entitled "RELEASABLE CATHETER HUB RETAINER". In some embodiments, the catheter system 10 may include one or more features of the safety catheter assembly described in U.S. Patent Application No. 62/808,133, filed February 20, 2019, entitled "IMPROVED COUPLING BETWEEN A TELESCOPING NEEDLE SHIELD AND A CATHETER ADAPTER".

In some embodiments, the needle shield device 12 may include a needle housing 30, which may be removably coupled to the catheter adapter 16. In some embodiments, the needle housing 30 may include an elongated body 32. In some embodiments, the needle shield device 12 may include a needle assembly 34, which may be slideably coupled to the needle housing 30.

In some embodiments, the needle assembly 34 may include an introducer needle 36, which may include sharp distal tip 38. In some embodiments, the introducer needle 36 may extend through the catheter 22 when the catheter system 10 is in an insertion position ready for insertion into vasculature of a patient, as illustrated, for example, in Figure 1A. In some embodiments, the needle assembly 34 may include a needle grip 40, which the user may grip and move proximally to withdraw the introducer needle 36 from the vasculature once placement of the catheter 22 within the vasculature is confirmed.

In some embodiments, placement of the catheter 22 within the vasculature may be confirmed via blood flashback. In some embodiments, in response to the introducer needle 36 being inserted into the vasculature of the patient, blood flashback may flow through the sharp distal tip 38 of the introducer needle 36 and out of a distal notch of the introducer needle 36 into a portion of the catheter system 10. For example, the blood flashback may flow through the sharp distal tip 38 and out of the distal notch into a space between an exterior surface of the introducer needle 36 and an interior surface of the catheter 22. In some embodiments, after the blood flashback is observed by the clinician, the introducer needle 36 may be withdrawn, and the needle assembly 34 and the needle housing 30 may be removed from the catheter adapter 16.

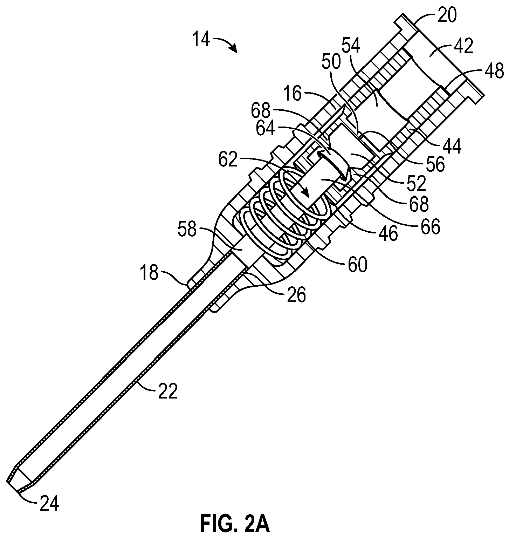

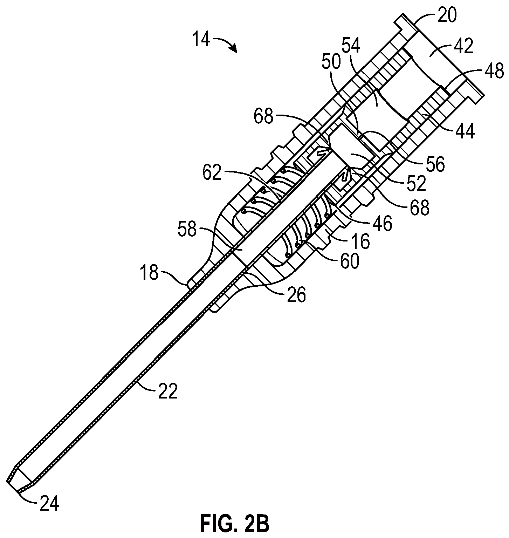

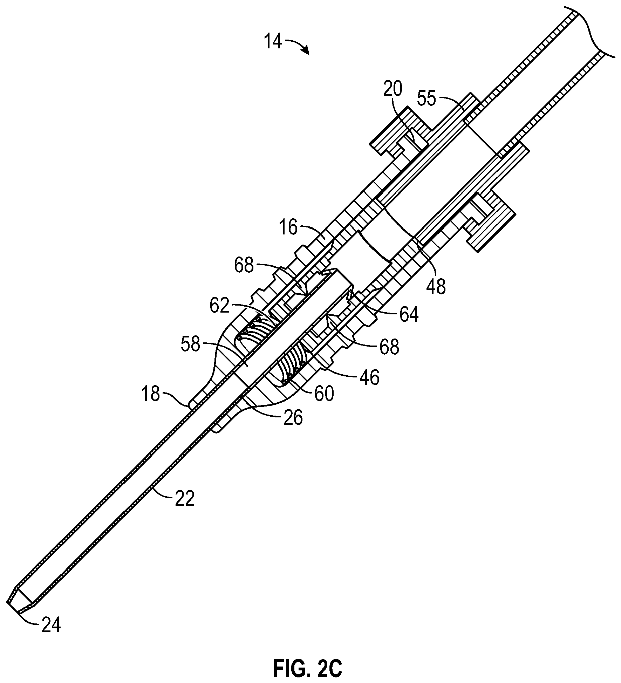

Referring now to Figures 2A-2C, the catheter assembly 14 includes the catheter adapter 16, which may include the distal end 18, the proximal end 20, and a lumen 42 extending through the distal end 18 of the catheter adapter 16 and the proximal end 20 of the catheter adapter 16.

The catheter assembly 14 includes a septum 44, disposed within the lumen 42. In some embodiments, the septum 44 may include a distal end 46, a proximal end 48, and a barrier 50 disposed between the distal end 46 of the septum 44 and the proximal end 48 of the septum 44. In some embodiments, the barrier 50 may divide an interior of the septum 44 into a distal cavity 52 and a proximal cavity 54. In some embodiments, the barrier 50 may prevent fluid from flowing between the distal cavity 52 and the proximal cavity 54 when the barrier 50 is closed. In some embodiments, the barrier 50 may include a slit 56. In some embodiments, the septum 44 may be configured to move from a proximal position to a distal position in response to insertion of a medical device 55 into the proximal end 20 of the catheter adapter 16.

The catheter assembly 14 includes a septum actuator 58, which may be fixed within the lumen 42. The septum actuator 58 is configured to penetrate the slit 56 in response to movement of the septum 44 from the proximal position to the distal position. In some embodiments, the septum 44 may include a substantially H-shaped cross-section.

The catheter assembly 14 includes a spring 60, disposed within the lumen 42 distal to the septum 44. The spring 60 is configured to return the septum 44 from the distal position to the proximal position in response to removal of the medical device 55 from the proximal end 20 of the catheter adapter 16. Thus, in some embodiments, the spring 60 may facilitate multiple uses of the catheter assembly 14 and the septum 44, including multiple infusions and/or blood withdrawals via multiple connections of the medical device 55 to the catheter adapter 16. In some embodiments, the spring 60 may be compressed in response to movement of the septum 44 to the distal position. In some embodiments, the spring 60 may be decompressed in response to movement of the septum 44 to the proximal position.

In some embodiments, the spring 60 may surround the septum actuator 58, as illustrated, for example, in Figure 2A. In some embodiments, the spring 60 may include a coil, as illustrated, for example in Figure 2A, which may surround the septum actuator 58. In some embodiments, the coil may be helical. In some embodiments, the coil may be constructed of metal or another suitable material. In some embodiments, the coil may be constructed of an elastomer. For example, the coil and/or the septum 44 may be constructed of silicon, which may provide improved mechanical properties, or another suitable material. In some embodiments, the coil and the septum 44 may be monolithically formed as a single unit. In some embodiments, a proximal end of the spring 60 may be coupled to the septum 44 and/or a distal end of the spring 60 may be coupled to the catheter adapter 16.

In some embodiments, the spring 60 may include a wave spring, which may be coiled and/or may surround the septum actuator 58. In some embodiments, the wave spring may be made from a coiled wire that includes waves to give the coiled wire a spring effect. In some embodiments, the wave spring may be constructed of metal or another suitable material. In some embodiments, the wave spring may be constructed of an elastomer. For example, the wave spring and/or the septum 44 may be constructed of silicon, which may provide improved mechanical properties, or another suitable material. In some embodiments, the wave spring and the septum 44 may be monolithically formed as a single unit. In some embodiments, a proximal end of the spring 60 may be coupled to the septum 44 and/or a distal end of the spring 60 may be coupled to the catheter adapter 16.

The septum actuator 58 includes a tubular body 62. In some embodiments, the tubular body 62 may be generally cylindrical or tapered. The septum actuator 58 includes an annular lip 64 extending outwardly from an outer surface 66 of the tubular body 62. The annular lip 64 extends distally from the outer surface 66 of the tubular body 62. In some embodiments, the annular lip 64 may be disposed at a proximal end of the septum actuator 58. In some embodiments, the annular lip 64 may include a generally truncated cone shape, as illustrated, for example, in Figure 2A. In some embodiments, the annular lip 64 or the generally truncated cone shape may facilitate securement of the septum 44 in the proximal position prior to insertion of the medical device 55 into the proximal end 20 of the catheter adapter 16, providing resistance to distal movement of the septum 44.

The inner surface of the septum 44 forming the distal cavity 52 includes one or more protrusions 68. In some embodiments, the one or more protrusions 68 may be annular. In some embodiments, the annular lip 64 may be configured to pass the one or more protrusions 68 in response to the septum being moved from the proximal position to the distal position. In some embodiments, the annular lip 64 may be configured to contact the one or more protrusions 68 when the septum 44 is moved from the proximal position to the distal position. In some embodiments, the one or more protrusions 68 may contact the tubular body 62 or the one or more protrusions 68 may not contact the tubular body 62.

In some embodiments, the one or more protrusions 68 and/or the annular lip 64 may provide resistance to the septum 44 returning from the distal position to the proximal position in response to removal of the medical device 55. In further detail, the one or more protrusions 68 may contact or catch on the annular lip 64 when the septum 44 moves towards the proximal position from the distal position and/or the annular lip 64 may contact or catch on the barrier 50 when the septum 44 moves towards the proximal position from the distal position. However, the spring 60 may overcome the resistance to facilitate movement of the septum 44 from the distal position to the proximal position. In this way, in some embodiments, the spring 60 may facilitate multiple uses of the catheter assembly 14 and the septum 44.

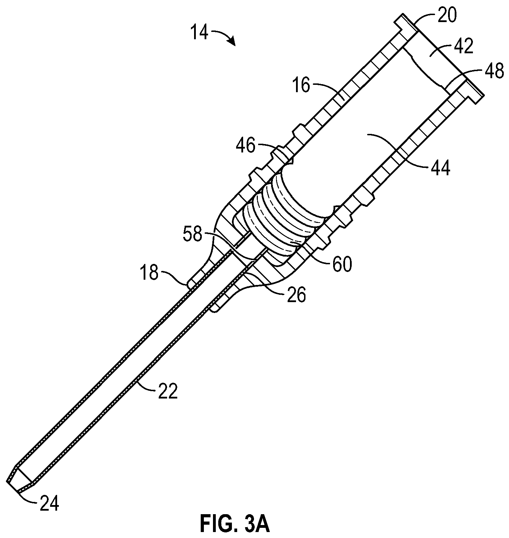

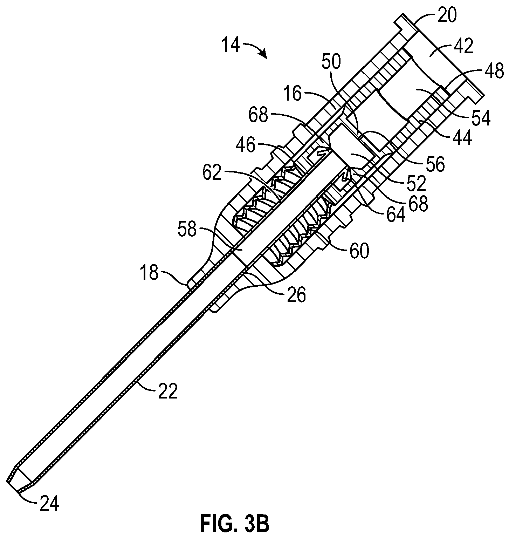

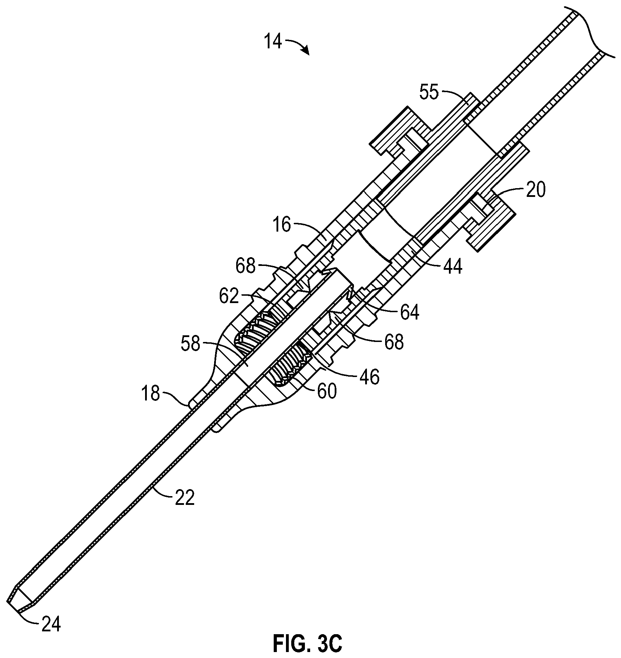

Referring now to Figures 3A-3C, in some embodiments, the spring 60 and/or the septum 44 may be constructed of an elastomer. For example, the spring 60 and/or the septum 44 may be constructed of silicon, which may provide improved mechanical properties, or another suitable material. In some embodiments, the spring 60 and the septum 44 may be monolithically formed as a single unit. In some embodiments, the spring 60 may surround the septum actuator 58.

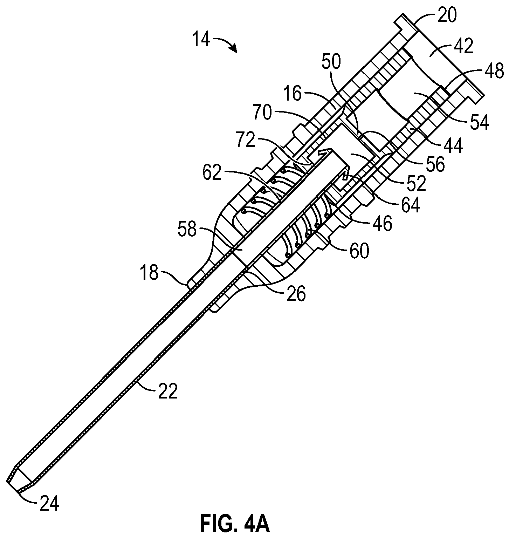

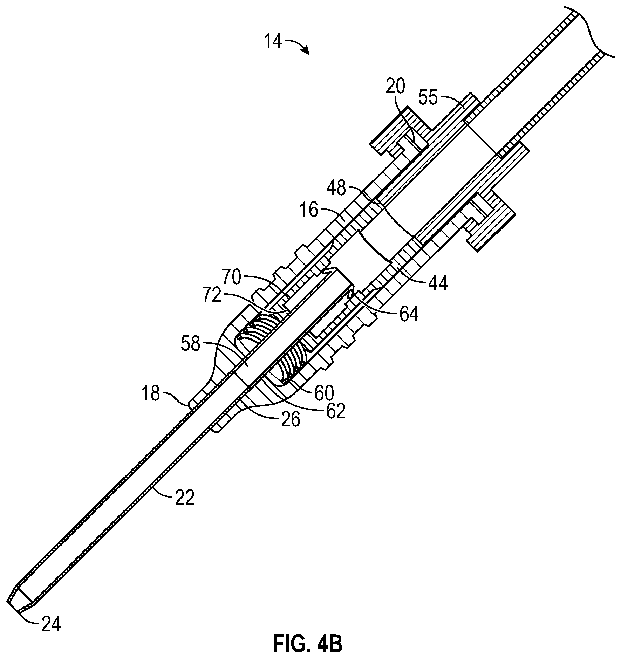

Referring now to Figures 4A-4B, in some embodiments, a portion 70 of the inner surface of the septum 44 forming the distal cavity 52 may be smooth. In these and other embodiments, the inner surface of the septum 44 forming the distal cavity 52 may not include the one or more protrusions 68. In some embodiments, the portion 70 of the inner surface may be distal to the barrier 50. In some embodiments, removal of the one or more protrusions 68 may facilitate return of the septum 44 from the proximal position to the distal position and multiple uses of the septum 44.

In some embodiments, the portion 70 of the inner surface may be aligned with or proximal to the annular lip 64 when the septum 44 is disposed in the proximal position. In some embodiments, the annular lip 64 may be spaced apart from the portion 70 of the inner surface of the septum 44 such that the annular lip 64 does not contact the portion 70 of the inner surface when the septum 44 is moved from the proximal position to the distal position. In some embodiments, the portion 70 of the inner surface may be proximate and/or proximal to an opening 72 of the distal end of the septum 44.

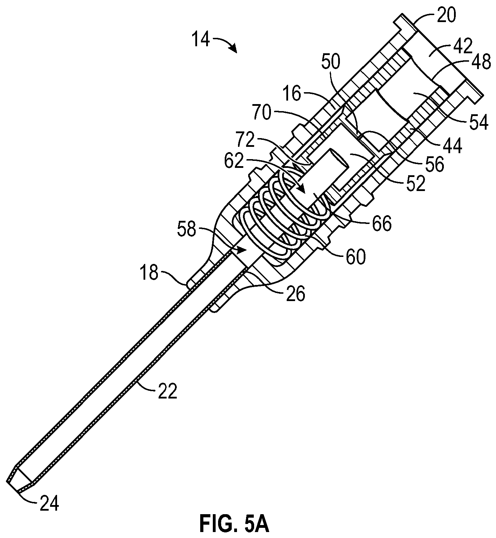

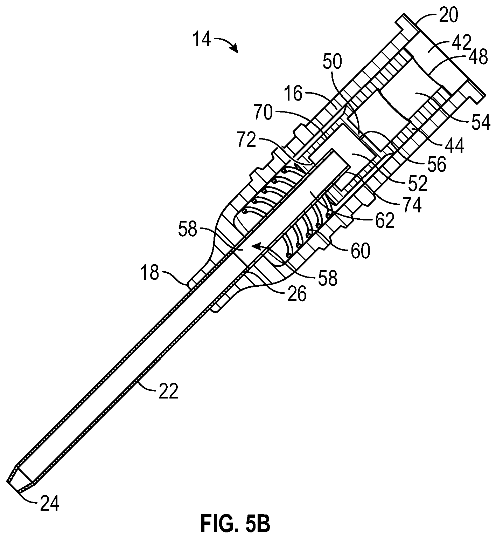

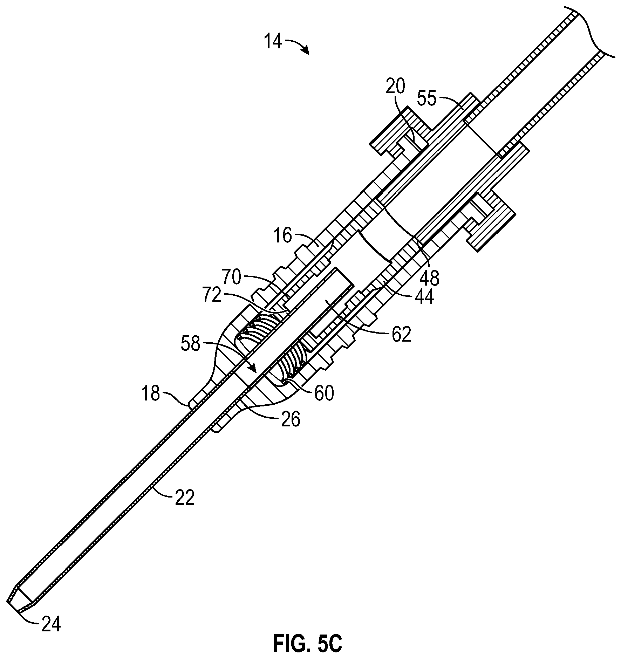

Referring now to Figures 5A-5C, in some embodiments, an outer surface of a proximal end 74 of the septum actuator 58 may be smooth and may not include the annular lip 64. In further detail, in some embodiments, an outer surface of the tubular body 62 may be smooth and may not include the annular lip 64. In some embodiments, removal of the annular lip 64 may facilitate return of the septum 44 from the proximal position to the distal position and multiple uses of the septum 44. In some embodiments, an inner surface of the septum 44 forming the distal cavity 52 may include the one or more protrusions 68. In other embodiments, the portion 70 of the inner surface of the septum 44 forming the distal cavity 52 may be smooth.

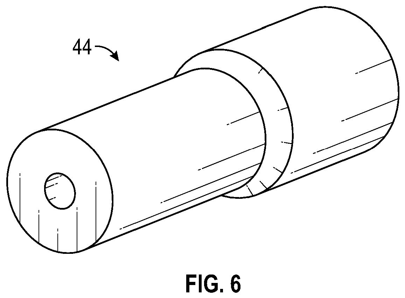

Referring now to Figure 6, an upper perspective view of the septum 44 is illustrated, according to some embodiments. In some embodiments, an outer surface of the septum 44 may be generally cylindrical.

All examples and conditional language recited herein are intended for pedagogical objects to aid the reader in understanding the invention and the concepts contributed by the inventor to furthering the art, and are to be construed as being without limitation to such specifically recited examples and conditions. Although embodiments of the present inventions have been described in detail, it should be understood that the various changes, substitutions, and alterations could be made hereto without departing from the scope of the invention as defined in the appended claims.

## Claims

1. A catheter assembly (14), comprising:
a catheter adapter (16), comprising a distal end (18), a proximal end (20), and a lumen (42) extending through the distal end and the proximal end;
a septum (44) disposed within the lumen, wherein the septum comprises a distal end (46), a proximal end (48), and a barrier (50) disposed between the distal end of the septum and the proximal end of the septum, wherein the barrier divides an interior of the septum into a distal cavity (52) and a proximal cavity (54), wherein the barrier comprises a slit (56), wherein the septum is configured to move from a proximal position to a distal position in response to insertion of a medical device into the proximal end of the catheter adapter;
a spring (60) disposed within the lumen distal to the septum, wherein the spring is configured to return the septum from the distal position to the proximal position in response to removal of the medical device from the proximal end of the catheter adapter, and
a septum actuator (58) fixed within the lumen and comprising a tubular body (62),
**characterized in that**
the septum actuator comprises an annular lip (64) extending outwardly from an outer surface (66) of the tubular body, wherein the annular lip is disposed at a proximal end of the septum actuator, wherein the septum actuator is configured to penetrate the slit in response to movement of the septum from the proximal position to the distal position,
wherein an inner surface of the septum forming the distal cavity comprises a protrusion (68), wherein the annular lip is configured to pass the protrusion in response to the septum being moved from the proximal position to the distal position.

2. The catheter assembly of claim 1, wherein the spring surrounds the septum actuator.

3. The catheter assembly of claim 1, wherein the spring and the septum are constructed of an elastomer.

4. The catheter assembly of claim 1, wherein the spring and the septum are constructed of silicone.

5. The catheter assembly of claim 1, wherein the spring and the septum are monolithically formed as a single unit.

6. The catheter assembly of claim 1, wherein an outer surface (66) of the tubular body is smooth.

7. The catheter assembly of claim 1, wherein the annular lip comprises a generally truncated cone shape.

8. The catheter assembly of claim 1, wherein a portion (70) of an inner surface of the septum forming the distal cavity is smooth, wherein the portion is distal to the barrier, wherein the portion is aligned with or proximal to the annular lip when the septum is disposed in the proximal position.

9. The catheter assembly of claim 1, wherein the spring comprises a coil, wherein the coil surrounds the septum actuator, wherein the coil comprises a helical shape or a plurality of waves.

10. The catheter assembly of claim 9, wherein the coil is constructed of metal.

11. The catheter assembly of claim 1, wherein a portion (70) of the inner surface of the septum is smooth, wherein the portion is distal to the barrier, wherein the portion is aligned with or proximal to the annular lip when the septum is disposed in the proximal position.

12. The catheter assembly of claim 11, wherein the spring surrounds the septum actuator.

13. The catheter assembly of claim 12, wherein the spring and the septum are monolithically formed as a single unit.

14. The catheter assembly of claim 13, wherein the spring and the septum are constructed of an elastomer.

15. The catheter assembly of claim 14, wherein the annular lip comprises a generally truncated cone shape.

16. The catheter assembly of claim 11, wherein the spring comprises a coil, wherein the coil surrounds the septum actuator.

## Patentansprüche

1. Katheteranordnung (14) mit:
einem Katheteradapter (16) mit einem distalen Ende (18), einem proximalen Ende (20), und einem Lumen (42), das sich durch das distale Ende und das proximale Ende erstreckt;
einem Septum (44), das in dem Lumen angeordnet ist, wobei das Septum ein distales Ende (46), ein proximales Ende (48), und eine Sperre (50) aufweist, die zwischen dem distalen Ende des Septums und dem proximalen Ende des Septums angeordnet ist, wobei die Sperre einen Innenraum des Septums in einen distalen Hohlraum (52) und einen proximalen Hohlraum (54) unterteilt, wobei die Sperre einen Schlitz (56) aufweist, wobei das Septum dazu ausgebildet ist, sich in Reaktion auf das Einführen einer medizinischen Vorrichtung in das proximale Ende des Katheteradapters von einer proximalen Position zu einer distalen Position zu bewegen;
einer Feder (60), die distal des Septums in dem Lumen angeordnet ist, wobei die Feder dazu ausgebildet ist, das Septum in Reaktion auf das Entfernen der medizinischen Vorrichtung aus dem proximalen Ende des Katheteradapters von der distalen Position in die proximale Position zurückzustellen; und
einem Septumaktuator (58), der in dem Lumen befestigt ist und einen rohrförmigen Körper (62) aufweist,
**dadurch gekennzeichnet,**
**dass** der Septumaktuator eine ringförmige Lippe (64) aufweist, die sich von einer Außenfläche (66) des rohrförmigen Körpers nach außen erstreckt, wobei die ringförmige Lippe an einem proximalen Ende des Septumaktuators angeordnet ist, wobei der Septumaktuator dazu ausgebildet ist, den Schlitz in Reaktion auf eine Bewegung des Septums von der proximalen Position in die distale Position zu durchdringen,
wobei eine Innenfläche des Septums, die den distalen Hohlraum bildet, einen Vorsprung (68) aufweist, wobei die ringförmige Lippe dazu ausgebildet ist, den Vorsprung in Reaktion auf das Bewegen des Septums von der proximalen Position in die distale Position zu passieren.

2. Katheteranordnung nach Anspruch 1, bei welcher die Feder den Septumaktuator umgibt.

3. Katheteranordnung nach Anspruch 1, bei welcher die Feder und das Septum aus einem Elastomer gebildet sind.

4. Katheteranordnung nach Anspruch 1, bei welcher die Feder und das Septum aus Silikon gebildet sind.

5. Katheteranordnung nach Anspruch 1, bei welcher die Feder und das Septum monolithisch als eine einzelne Einheit gebildet ist.

6. Katheteranordnung nach Anspruch 1, bei welcher eine Außenseite (66) des rohrförmigen Körpers glatt ist.

7. Katheteranordnung nach Anspruch 1, bei welcher die ringförmige Lippe im Allgemeinen kegelstumpfförmig ist.

8. Katheteranordnung nach Anspruch 1, bei welcher ein Bereich (70) einer Innenfläche des Septums, welche den distalen Hohlraum bildet, glatt ist, wobei sich der Bereich distal der Sperre befindet, wobei der Bereich mit der ringförmigen Lippe ausgerichtet oder zu dieser proximal ist, wenn das Septum in der proximalen Position angeordnet ist.

9. Katheteranordnung nach Anspruch 1, bei welcher die Feder eine Spule aufweist, wobei die Spule den Septumaktuator umgibt, wobei die Spule eine Schraubenlinienform oder mehrere Wellen aufweist.

10. Katheteranordnung nach Anspruch 9, bei welcher die Spule aus Metall gebildet ist.

11. Katheteranordnung nach Anspruch 1, bei welcher ein Bereich (70) der Innenfläche des Septums glatt ist, wobei der Bereich sich distal der Sperre befindet, wobei der Bereich mit der ringförmigen Lippe ausgerichtet oder zu dieser proximal ist, wenn das Septum in der proximalen Position angeordnet ist.

12. Katheteranordnung nach Anspruch 11, bei welcher die Feder den Septumaktuator umgibt.

13. Katheteranordnung nach Anspruch 12, bei welcher die Feder und das Septum monolithisch als eine einzelne Einheit gebildet sind.

14. Katheteranordnung nach Anspruch 13, bei welcher die Feder und das Septum aus einem Elastomer gebildet sind.

15. Katheteranordnung nach Anspruch 14, bei welcher die ringförmige Lippe im Allgemeinen kegelstumpfförmig ist.

16. Katheteranordnung nach Anspruch 11, bei welcher die Feder eine Spule aufweist, wobei die Spule den Septumaktuator umgibt.

## Revendications

1. Ensemble cathéter (14), comprenant :
un adaptateur de cathéter (16), comprenant une extrémité distale (18), une extrémité proximale (20) et une lumière (42) s'étendant à travers l'extrémité distale et l'extrémité proximale ;
un septum (44) disposé à l'intérieur de la lumière, dans lequel le septum comprend une extrémité distale (46), une extrémité proximale (48), et une barrière (50) disposée entre l'extrémité distale du septum et l'extrémité proximale du septum, dans lequel la barrière divise un intérieur du septum en une cavité distale (52) et une cavité proximale (54), dans lequel la barrière comprend une fente (56), dans lequel le septum est configuré pour se déplacer d'une position proximale à une position distale en réponse à l'insertion d'un dispositif médical dans l'extrémité proximale de l'adaptateur de cathéter ;
un ressort (60) disposé à l'intérieur de la lumière distal par rapport au septum, dans lequel le ressort est configuré pour ramener le septum de la position distale à la position proximale en réponse au retrait du dispositif médical depuis l'extrémité proximale de l'adaptateur de cathéter, et
un actionneur de septum (58) fixé à l'intérieur de la lumière et comprenant un corps tubulaire (62),
**caractérisé en ce que**
l'actionneur de septum comprend une lèvre annulaire (64) s'étendant vers l'extérieur à partir d'une surface extérieure (66) du corps tubulaire, dans lequel la lèvre annulaire est disposée au niveau d'une extrémité proximale de l'actionneur de septum, dans lequel l'actionneur de septum est configuré pour pénétrer dans la fente en réponse à un déplacement du septum de la position proximale à la position distale,
dans lequel une surface intérieure du septum formant la cavité distale comprend une saillie (68), dans lequel la lèvre annulaire est configurée pour laisser passer la saillie en réponse au déplacement du septum de la position proximale à la position distale.

2. Ensemble cathéter selon la revendication 1, dans lequel le ressort entoure l'actionneur de septum.

3. Ensemble cathéter selon la revendication 1, dans lequel le ressort et le septum sont réalisés en un élastomère.

4. Ensemble cathéter selon la revendication 1, dans lequel le ressort et le septum sont réalisés en silicone.

5. Ensemble cathéter selon la revendication 1, dans lequel le ressort et le septum sont formés de manière monolithique sous la forme d'une unité unique.

6. Ensemble cathéter selon la revendication 1, dans lequel une surface extérieure (66) du corps tubulaire est lisse.

7. Ensemble cathéter selon la revendication 1, dans lequel la lèvre annulaire comprend une forme de cône généralement tronqué.

8. Ensemble cathéter selon la revendication 1, dans lequel une partie (70) d'une surface interne du septum formant la cavité distale est lisse, dans lequel la partie est distale par rapport à la barrière, dans lequel la partie est alignée avec ou proximale par rapport à la lèvre annulaire lorsque le septum est disposé dans la position proximale.

9. Ensemble cathéter selon la revendication 1, dans lequel le ressort comprend une bobine, dans lequel la bobine entoure l'actionneur de septum, dans lequel la bobine comprend une forme hélicoïdale ou une pluralité d'ondes.

10. Ensemble cathéter selon la revendication 9, dans lequel la bobine est réalisée en métal.

11. Ensemble cathéter selon la revendication 1, dans lequel une partie (70) d'une surface interne du septum est lisse, dans lequel la partie est distale par rapport à la barrière, dans lequel la partie est alignée avec ou proximale par rapport à la lèvre annulaire lorsque le septum est disposé dans la position proximale.

12. Ensemble cathéter selon la revendication 11, dans lequel le ressort entoure l'actionneur de septum.

13. Ensemble cathéter selon la revendication 12, dans lequel le ressort et le septum sont formés de manière monolithique sous la forme d'une unité unique.

14. Ensemble cathéter selon la revendication 13, dans lequel le ressort et le septum sont réalisés en un élastomère.

15. Ensemble cathéter selon la revendication 14, dans lequel la lèvre annulaire comprend une forme de cône généralement tronqué.

16. Ensemble cathéter selon la revendication 11, dans lequel le ressort comprend une bobine, dans lequel la bobine entoure l'actionneur de septum.
